# EUROPEAN PATENT SPECIFICATION

(11) **EP 1 554 266 B1**
(45) Date of publication and mention of the grant of the patent: **22.03.2006**
(21) Application number: 03808838.1
(22) Date of filing: 13.10.2003
(51) Int. Cl.: C07D 333/08, C07D 333/16

(54) **PROCESS FOR PREPARING 2,5-DISUBSTITUTED 3-ALKYLTHIOPHENES**
VERFAHREN ZUR HERSTELLUNG 2,5-DISUBSTITUIERTER 3-ALKYLTHIOPHENEN
PROCEDE D'ELABORATION DE 3-ALKYLTHIOPHENES A DISUBSTITUTION EN 2,5

(30) Priority: 14.10.2002 IT MI20020021
(43) Date of publication of application: 20.07.2005
(73) Proprietor: Clariant Life Science Molecules (Italia) S.p.A., 20124 Milano (IT)
(72) Inventor: FRIGERIO, Marco, I-20147 Milano (IT); BERTOLINI, Giorgio, I-20099 Sesto San Giovanni (Milano) (IT)
(74) Representative: Dünnwald, Dieter
(86) International application number: PCT/IB2003/004498
(87) International publication number: WO 2004/035562

(56) References cited:
- WO-A-99/61435
- US-A- 6 103 708

## Description

The present invention relates to a novel process for preparing 2,5-disubstituted 3-alkylthiophenes and more particularly to a process for preparing them that comprises an acylation reaction in position 5 of 2-substituted 3-alkylthiophenes.

### PRIOR ART

2,5-Disubstituted 3-alkylthiophenes, of general formula 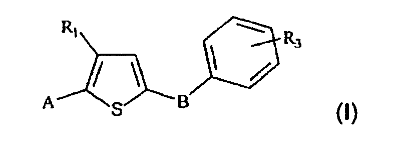 and in particular 2,3-dimethyl-5-benzylthiophene of formula 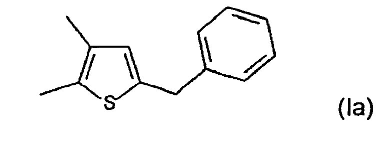 (R₁=A=CH₃, B=CH₂, R₃=H), are useful synthetic intermediates.

For example, in US patents 6 121 271 and 6 103 708 and in international patent applications WO 99/61435 and WO 99/58522, compound (Ia) is used as an intermediate in the preparation of medicinal products for treating metabolic disorders related to insulin resistance or hyperglycemia.

Two different processes for preparing compound (Ia) are also described in the abovementioned documents.

More particularly, international patent application WO 99/61435 illustrates the synthesis of compound (Ia) according to the sequence given below:

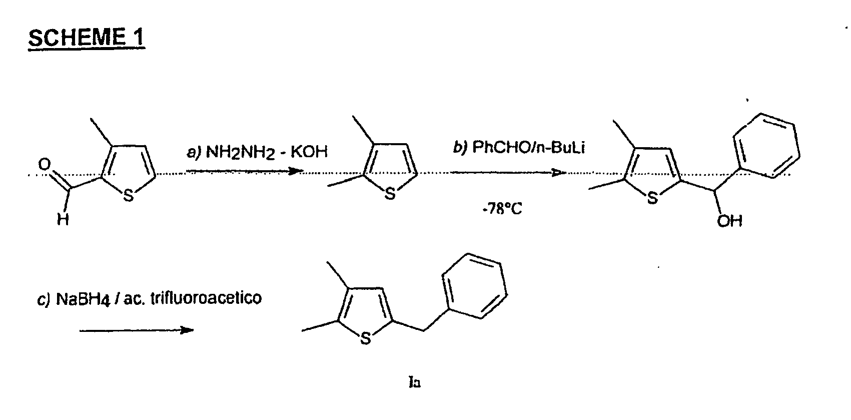

As may be seen, this synthesis involves the reduction of 3-methylthiophene-2-carboxaldehyde (step *a*) to give 2,3-dimethylthiophene, its functionalization in position 5 (step *b*) and, finally, subsequent reduction of the intermediate alcohol (step *c*) to give the desired compound (Ia).

However, this process is difficult to apply industrially since it involves the use of n-BuLi, which is a notoriously flammable reagent that requires an inert atmosphere, an anhydrous medium and a low temperature (-78°C).

Another non-neglible drawback is represented by the need to use, in the condensation with benzaldehyde (step b), an intermediate 2,3-dimethylthiophene of high purity in order to minimize the possible side reactions and thus to obtain the desired product (Ia) in acceptable overall yields, which in this case would be about 67%, and with a sufficient degree of purity for subsequent direct synthetic use. The starting material for the process, i.e. 3-methylthiophene-2-carboxaldehyde, is, however; commercially available with a relatively poor degree of purity, generally of about 90%, and consequently the impure 2,3-dimethylthiophene obtained by reducing it cannot be used in unpurified form in the subsequent step, but rather is always subjected to purification; in particular, being a an oil, it is purified by chromatography, which is definitely not very method practicable from an industrial point of view.

Similarly, the final compound (Ia), which is a high-boiling liquid, is difficult to isolate and therefore another possible purification at level, by means of chromatography or distillation, would be relatively problematic on an industrial scale.

The second process for preparing compound (Ia), described in patents US 6 121 271 and US 6 103 708 and in international patent application WO 99/58522, may be represented according to the following scheme:

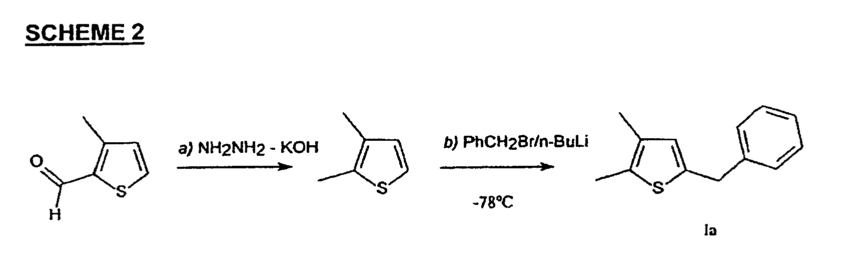

In this case also, the same drawbacks described above may be encountered, i.e. the use of n-BuLi, which is difficult to work with industrially, and the use of two successive chromatographies for the purification of the intermediate 2,3-dimethylthiophene and the final product (Ia), which are both liquids at room temperature.

We have now found a novel process for preparing 2,5-disubstituted 3-alkylthiophenes, in particular 2,3-dimethyl-5-benzylthiophene, which is significantly advantageous in industrial terms since it does not use reagents that are relatively difficult to work with such as n-BuLi, it does not require anhydrous conditions or inert atmospheres, and it appreciably simplifies the purification stages. This process makes it possible above all to use commercially available technical-grade starting materials directly, and to obtain the final product (I) in yields comparable to those for the known preparations but with high purity, entirely avoiding the use of chromatographic methods or distillation techniques, both on the intermediates and on the final product. The reason for this is that these final products generally have a purity that allows them to be used directly in subsequent processes without further purification.

### SUMMARY OF THE INVENTION

One subject of the present invention is therefore a process for preparing 2,5-disubstituted 3-alkylthiophenes of formula 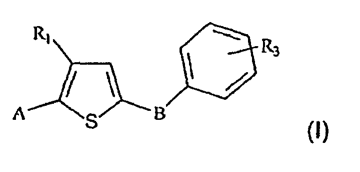 in which
- A: represents a CH₃, R₂CH₂, HOCH₂ or R₂CH(OH)- group,
- B: represents a CHOH or CH₂ group,
- R₁: represents H or a C₁-C₅ alkyl group,
- R₂: represents a C₁-C₅ alkyl group,
- R₃: represents H or a C₁-C₅ alkyl group or a C₁-C₅ haloalkyl group, preferably CF₃, or a halogen chosen from fluorine, chlorine and bromine,
which comprises:
(*a*) the reaction of a compound of formula 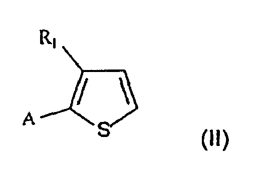 in which
   - A: represents a CHO, CH₃, R₂CH₂ or R₂-CO- group, and
   - R₁ and R₂: have the meanings given above;
   with a compound of formula 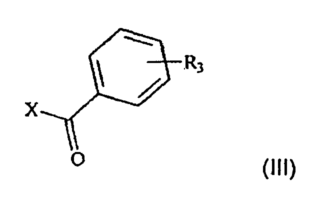 in which
   - X: represents OH, halogen or a group of formula 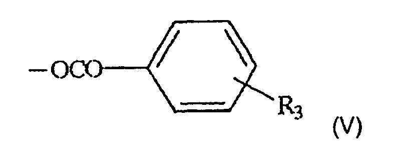
   or a group of formula

   -OCOOR₄ (VI)

   in which
   - R₄: represents a C₁-C₅ alkyl, an optionally substituted benzyl or an optionally substituted aryl, and
   - R₃: has the meanings given above;
   to give a compound of formula 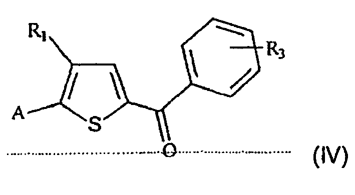 in which
   A, R₁, R₂ and R₃ have the meanings given above; and
(*b*) the reduction of the compound of formula (IV) thus obtained to give the compound of formula (I).

### DESCRIPTION OF THE INVENTION

The process that is the subject of the present invention comprises the acylation reaction (*a*) of the compound of formula II and the subsequent reduction (*b*) of the acyl intermediate of formula IV to give the desired compound I.

Generally, the starting compound of formula II is commercially available or may be prepared according to processes known to those skilled in the art. For example, in the case where it is desired to use the preferred compound IIb (R₁=A=CH₃) as starting compound, it may be prepared, in accordance with that described in the abovementioned prior art, by reduction, with hydrazine and potassium hydroxide, of the commercially available compound IIa (R₁=CH₃, A=CHO). As disclosed previously, an appreciable advantage of the present process is the direct use of the commercial compound IIa (purity of about 90%) in the reduction reaction to give IIb or, alternatively, in the acylation reaction (a) to give compound IVa (R₁=CH₃, A=CHO, R₃=H).

The acylation reaction referred to in point (*a*) is generally performed under the standard conditions of Friedel-Crafts acylations.

The acylating compound of formula III may be a carboxylic acid (X=OH), an acid halide (X=halogen), preferably chloride (X=CI), a symmetrical anhydride (X=V) or a mixed anhydride (X=VI).

Preferred mixed anhydrides are those in which R₄ represents methyl, ethyl, i-butyl or benzyl, more preferably methyl or ethyl.

Acyl halides are preferred acylating agents, and benzoyl chloride is even more preferred (IIIa, X=Cl, R₃=H).

The catalysts that may be used in this reaction are generally Lewis acids, for instance AlCl₃, SbF₅, FeCl₃, TiCl₄ or BF₃, preferably AlCl₃.

Suitable solvents are those commonly used by those skilled in the art for reactions of this type, i.e. chlorinated solvents such as methylene chloride, chloroform or trichloroethane, or deactivated aromatic solvents such as nitrobenzene, preferably methylene chloride.

In the acylation reaction (*a*), a molar ratio of compound III/Lewis acid/compound II generally of between 0.9-1.5/0.9-1.5/1 and preferably of about 1/1/1 per unit of compound II is used.

The second step of the present process (step b) involves the reduction of the intermediate of formula IV to give the desired product of formula I.

Depending on the structure of the intermediate IV and of the final compound I, this reduction may be performed by means of a single reductive treatment (*b*) or, preferably, by means of a first reduction reaction (*b*₁), optionally followed by a second reduction reaction (*b*₂).

In the case where it is desired to obtain the completely reduced compounds of formula I directly, a single reductive treatment of the compound of formula IV will be performed to give the compound of formula I in which A= CH₃ or R₂CH₂ and B=CH₂.

In this case, a reduction reaction may be performed under the standard conditions for reducing C=O groups to CH₂ groups, for example by using a borohydride in the presence of a strong acid, for instance trifluoroacetic acid, methanesulphonic acid or sulphuric acid, preferably sodium borohydride or sodium cyanoborohydride in the presence of trifluoroacetic acid, or zinc iodide or trimethylsilyl chloride or trialkylsilanes in the presence of trifluoroacetic acid.

Alternatively, the process may be performed by means of a first reduction reaction (*b*₁) of the compound of formula 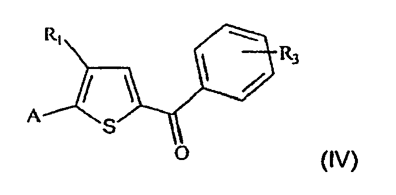 in which A represents a CHO, CH₃, R₂CH₂ or R₂-CO- group, to give the hydroxylated intermediate compound of formula 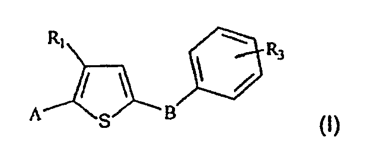 in which A represents a CH₃, R₂CH₂, HOCH₂ or R₂CH(OH)- group and B=CHOH, optionally followed by a second reduction reaction (b₂) of the hydroxylated intermediate of formula I as defined above, to give the final compound of formula 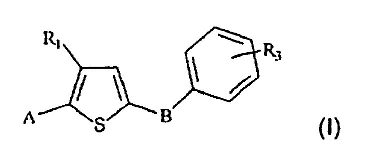 in which A represents a CH₃ or R₂CH₂ group and B=CH₂.

In the first step (*b*₁), suitable conditions may be used to perform the reduction of the C=O groups to CHOH groups, for instance treatment with metal hydrides such as sodium borohydride, lithium aluminium hydride or boranes, preferably with sodium borohydride in alkaline medium, or alternatively by treatment with aluminium isopropoxide (Meerwein-Ponndorf-Verley reaction).

The intermediate hydroxylated derivatives of formula I as defined above, preferably the compounds Ib (R₁=CH₃, A=CH₂OH, B=CHOH, R₃=H) or Ic (R₁=A=CH₃, B=CHOH, R₃=H) may then be subjected to the subsequent reduction step (*b*₂), directly or, preferably, after purification.

This purification is advantageously performed by crystallization, since the said hydroxylated intermediates are generally crystalline solids.

Suitable crystallization solvents are usually apolar solvents such as ethers, for example diethyl ether, diisopropyl ether or methyl tert-butyl ether, aromatic hydrocarbons, for example toluene or xylene, aliphatic hydrocarbons, for example hexane, heptane or cyclohexane, or mixtures of these solvents.

By then performing this preferred variant of the process, which involves a first reduction (CO->CHOH, *b*₁), the crystallization of the hydroxylated intermediate and its subsequent total reduction (CHOH-->CH₂ *b*₂), it is thus possible to perform the entire synthetic sequence of the present invention without the need for chromatographic purifications or distillations, which are drawbacks from an industrial point of view.

The hydroxylated compounds of formula I, preferably the abovementioned compounds Ib and Ic, which are preferably purified, may then be converted into the corresponding completely reduced compounds of formula I, preferably into compound Ia, by reduction (*b*₂) of the CHOH groups to CH₂ groups, for example by treatment with a borohydride in the presence of a strong acid, for instance trifluoroacetic acid, methanesulphonic acid or sulphuric acid, preferably with sodium borohydride or sodium cyanoborohydride and trifluoroacetic acid, or zinc iodide, or, surprisingly, by means of catalytic hydrogenation.

This latter reaction may be performed simply by treating the hydroxylated compound I, dissolved in a suitable solvent such as an alcohol, for instance methanol, ethanol or isopropanol, preferably methanol, or in mixtures of water and alcohols at a hydrogen pressure of between about 1 and 10 bar, at a temperature of between about 15 and 60°C, in the presence of a hydrogenation catalyst chosen from palladium and platinum, preferably palladium supported on an inert support such as carbon, alumina, silica or zeolites, preferably on carbon, in a neutral or acidic medium, without observing the foreseeable poisoning of the catalyst normally caused by sulphur-containing compounds, as are the thiophene derivatives prepared in the present process.

The general scheme of the process that is the subject of the present invention is given hereinbelow for greater clarity:

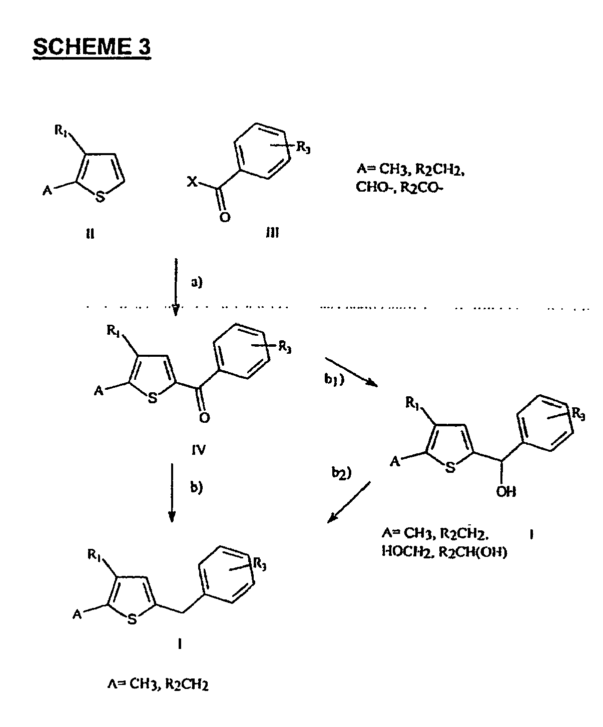

The possible variants of the reduction step *(b, b*_{*1*}*, b*_{*2*}*),* which are within the scope of the process according to the present invention, are shown in this scheme. The choice of the optimum sequence and of the most advantageous reduction conditions forms part of the normal capabilities of a person skilled in the art.

One preferred embodiment of the present invention is represented by the preparation of 2,3-dimethyl-5-benzylthiophene (Ia) according to the following scheme:

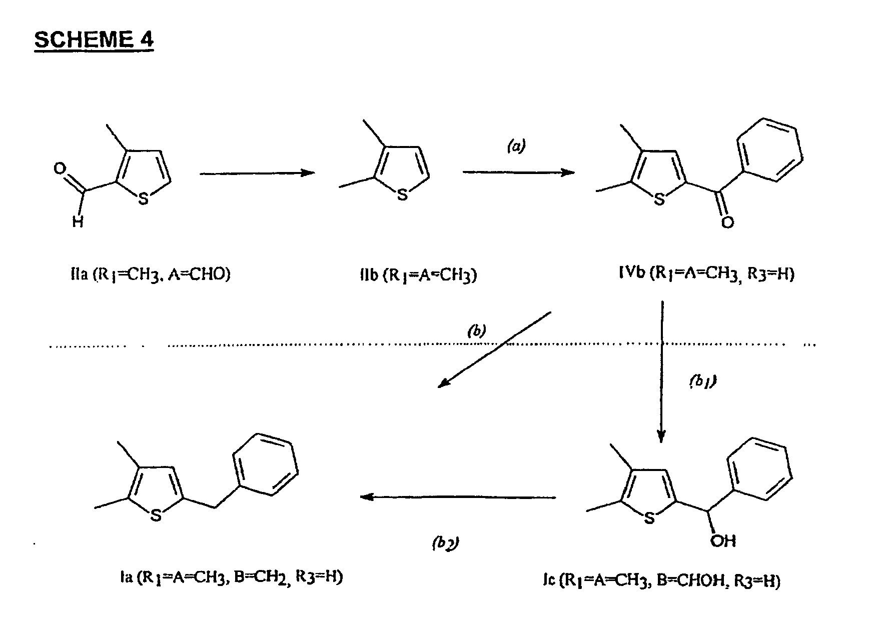

In particular, the product IIb may be prepared from the commercially available low-quality compound IIa, by reduction with hydrazine and sodium hydroxide, as described, for example, in WO 99/61435, and used in the next step without being isolated or purified.

Specifically, the acylation reaction (*a*) of compound IIb, preferably performed with benzoyl chloride and AlCl₃, may be performed by directly using its organic extraction solution, which is preferably methylenic, originating from the reduction of compound IIa.

Compound lVb thus obtained may be totally reduced (*b*), to give Ia, for example by reaction with sodium borohydride and trifluoroacetic acid or, preferably, subjected to two successive reduction reactions (*b*₁ and *b*₂) with formation of the hydroxylated intermediate Ic. This latter variant of the process is particularly preferred, since it allows the intermediate Ic to be purified by crystallization.

In particular, the first reduction reaction (*b*₁) is preferably performed with sodium borohydride and sodium hydroxide, while the second reduction with sodium borohydride and trifluoroacetic acid or by means of catalytic hydrogenation, preferably with sodium borohydride and trifluoroacetic acid. The intermediate Ic is generally crystallized from apolar solvents such as ethers, for example diethyl ether, diisopropyl ether or methyl tert-butyl ether, aromatic hydrocarbons, for example toluene or xylene, or aliphatic hydrocarbons, for example hexane, heptane or cyclohexane, or mixtures of these solvents, preferably from n-heptane.

The preferred process given in Scheme 4, in particular in its preferred variant, comprising the two successive reduction reactions *b*₁ and *b*₂ and the crystallization of the hydroxylated intermediate Ic makes it possible to prepare compound Ia in an overall yield that is comparable with that obtained according to the abovementioned known processes, but avoiding the reagents, reaction conditions and purification techniques of the prior art that are difficult to apply industrially.

In addition, as mentioned previously, the present process may be applied using the commercially available low-quality product IIa. Specifically, by using compound IIa with a GC purity of 90%, it is possiblre to obtain 2,3-dimethyl-5-benzylthiophene la with an HPLC purity of about 99%, without the need for chromatographic purifications, but merely by means of a simple crystallization of the intermediate alcohol Ic.

Finally, another preferred embodiment of the present invention is represented by the preparation of compound I according to the following scheme:

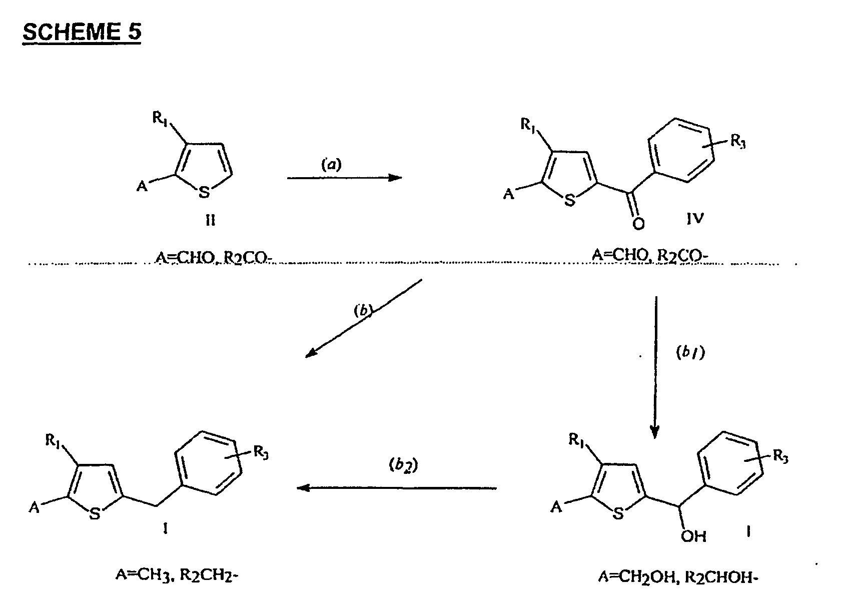

In this case, the product II, in which A represents CHO or R₂CO-, is acylated directly to give the intermediate IV which is then subjected to the simultaneous reduction of both the carbonyl groups.

By analogy with that described previously, this conversion may be performed by means of a single reduction reaction (*b*) or, preferably, two successive reduction reactions (*b*_{*1*} and *b*_{*2*}), with the possibility of purification of the intermediate hydroxylated compound by crystallization.

One particularly preferred embodiment of this process is the preparation of 2,3-dimethyl-5-benzylthiophene (Ia), from 3-methyl-2-thiophenecarboxaldehyde (IIa, A=CHO, R₁=CH₃) by acylation (a) with benzoyl chloride, reduction (*b*_{*1*}) of compound IVa (A=CHO, R₁=CH₃, R₃=H) to the diol Ib (A=CH₂OH, R₁=CH₃, R₃=H), purification thereof by crystallization and subsequent reduction (*b*_{*2*}) to give the final compound la (A=R₁=CH₃, R₃=H).

The examples that follow will now be given for the purpose of illustrating the present invention more clearly.

### EXPERIMENTAL SECTION

### EXAMPLE 1

### Preparation of 2,3-dimethylthiophene (IIb, R₁=A=CH₃)

3-Methyl-2-thiophenecarboxaldehyde (Aldrich, 90% GC purity) (40 g) was dissolved in diethylene glycol (144 ml) and hydrazine hydrate (62 ml) was added slowly. The reaction mixture was refluxed (about 126°C) for 30 minutes. The solution was cooled to 30-40°C and potassium hydroxide (46 g) was then added portionwise. The solution was then slowly heated to reflux and stirred at this temperature for one and a half hours.

The reaction mixture was cooled to room temperature and poured into cold water (1 L), acidified to pH 2 with concentrated hydrochloric acid (about 150 ml) and extracted with methylene chloride (2x100 ml). The combined organic phases were dried over anhydrous sodium sulphate. The solid was filtered off and washed with methylene chloride (20 ml) and the 2,3-dimethylthiophene solution was used directly without further purification (88.2% GC purity).

### EXAMPLE 2

### Preparation of 2,3-dimethyl-5-benzoylthiophene (IVb, R₁=A=CH₃, R₃=H)

Benzoyl chloride (37.2 ml) was dissolved in the methylenic solution containing about 35.6 g of 2,3-dimethylthiophene obtained from Example 1. This solution was added slowly at 0-5°C to a solution of anhydrous aluminium trichloride (42.7 g) in methylene chloride (100 ml) over 2-3 hours. The reaction mixture was stirred at 20-25°C for 2 hours and was then refluxed for one hour. After cooling to room temperature, the reaction mixture was poured into water (500 ml) at 0°C and stirred for 15 minutes. The two phases were separated and the organic phase was washed first with water (500 ml) and then with aqueous 30% sodium hydroxide solution (30 ml). The organic phase was evaporated under vacuum to give 64.5 g of 2,3-dimethyl-5-benzoylthiophene (94% yield over two steps) (88.8% GC purity).
¹H-NMR (300 MHz, DMSO) δ: 7.78 (d, 2H), 7.64 (t, 1H), 7.54 (t, 2H), 7.43 (s, 1H), 2.39 (s, 3H), 2.12 (s, 3H).

### EXAMPLE 3

### Preparation of 2,3-dimethyl-5-(α-hydroxybenzyl)thiophene (Ic, R₁=A=CH₃ B=CHOH. R₃=H)

Sodium borohydride (7.0 g) was dissolved in water (40 ml) and 30% sodium hydroxide (2 ml) and this solution was added slowly over about one hour to a solution of 2,3-dimethyl-5-benzoylthiophene (64.5 g) in methanol (250 ml) and 30% sodium hydroxide (3.5 ml). The solution was heated at 35°C for 6 hours and then refluxed for 2 hours. The reaction mixture was then cooled to 35-40°C and the methanol was evaporated off under vacuum. The residue was dissolved in water (150 ml), 30% sodium hydroxide (20 ml) and methylene chloride (150 ml). The two phases were separated and the aqueous phase was extracted with methylene chloride (50 ml). The organic phase was evaporated under vacuum and the crude product was dissolved in hot n-heptane (700 ml) and crystallized to give, after drying at 40°C, 46.0 g of 2,3-dimethyl-5-(α-hydroxybenzyl)thiophene (71% yield) (99.1% GC purity).
m.p. = 79-80°C,
¹H-NMR (300 MHz, DMSO) δ: 7.40-7.23 (m, 5H), 6.52 (s, 1H), 6.03 (s, 1H), 5.72 (s, 1 H), 2.21 (s, 3H), 1.99 (s, 3H).

### EXAMPLE 4

### Preparation of 2,3-dimethyl-5-benzylthiophene Ia, R₁=A=CH₃, B=CH₂, R₃=H)

2,3-Dimethyl-5-(α-hydroxybenzyl)thiophene (46.0 g) was dissolved in THF (1100 ml), followed by addition of sodium borohydride (23.9 g). The reaction mixture was cooled to 0°C and trifluoroacetic acid was added slowly over about two hours at 0-5°C. The reaction mixture was stirred at 0-5°C for 2 hours. A 30% solution of sodium hydroxide (150 ml) in water (300 ml) was added and the two phases were separated.

The organic phase was evaporated under vacuum and the residue was dissolved in methylene chloride. The salts were filtered off and the organic solution was washed with aqueous sodium chloride solution and evaporated under vacuum to give 40.5 g of 2,3-dimethyl-5-benzylthiophene as an oil (95% yield) (98.8% HPLC purity).
¹H-NMR (300 MHz, DMSO) δ: 7.32-7.20 (m, 5H), 6.54 (s, 1 H), 3.99 (s, 2H), 2.21 (s, 3H), 2.02 (s, 3H).

### EXAMPLE 5

### Preparation of 2,3-dimethyl-5-benzylthiophene Ia, R₁=A=CH₃, B=CH₂, R₃=H)

2,3-Dimethyl-5-(α-hydroxybenzyl)thiophene (46.0 g) was dissolved in methanol (750 ml), followed by addition of 5% palladium-on-charcoal containing 50% water (7.5 g on a dry basis). The reaction mixture was hydrogenated at 40-45°C and 6-7 bar for 24 hours, to give 26 g of 2,3-dimethyl-5-thiophene (61% yield).

## Claims

1. Process for preparing 2,5-disubstituted 3-alkylthiophenes of formula 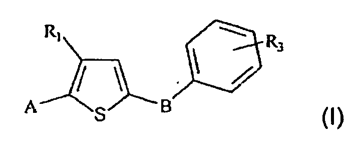 in which
A represents a CH₃, R₂CH₂, HOCH₂ or R₂CH(OH)- group,
B represents a CHOH or CH₂ group,
R₁ represents H or a C₁-C₅ alkyl group,
R₂ represents a C₁-C₅ alkyl group,
R₃ represents H or a C₁-C₅ alkyl group or a C₁-C₅ haloalkyl group, or a halogen chosen from fluorine, chlorine and bromine,
which comprises:
(*a*) the reaction of a compound of formula 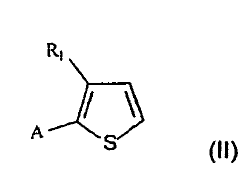 in which
A represents a CHO, CH₃, R₂CH₂ or R₂-CO- group, and
R₁ and R₂ have the meanings given above;
with a compound of formula 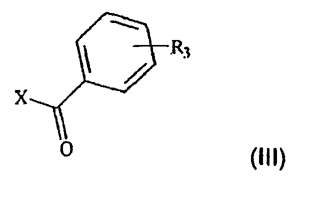 in which
X represents OH, halogen or a group of formula 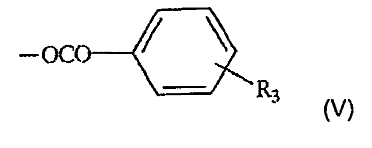
or a group of formula
-OCOOR₄ (VI)
in which
R₄ represents a C₁-C₅ alkyl, an optionally substituted benzyl or an optionally substituted aryl, and
R₃ has the meanings given above;
to give a compound of formula 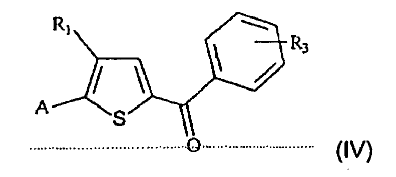 in which
A, R₁, R₂ and R₃ have the meanings given above; and
(*b*) the reduction of the compound of formula IV thus obtained to give the compound of formula I.

2. Process according to Claim 1 for the preparation of 2,5-disubstituted 3-alkylthiophenes of formula 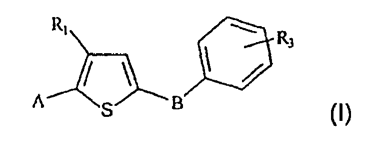 in which
A represents a CH₃ or R₂CH₂ group,
which comprises:
(*a*) the reaction of a compound of formula 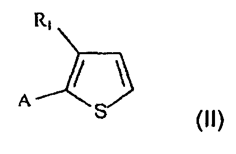 in which
A represents a CH₃ or R₂CH₂ group,
with a compound of formula 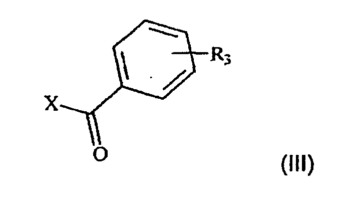 to give a compound of formula 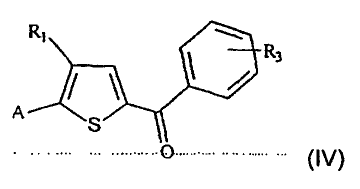 in which
A represents a CH₃ or R₂CH₂ group, and
(*b*) the reduction of the compound of formula IV thus obtained to give the compound of formula I.

3. Process according to Claim 1 for the preparation of 2,5-disubstituted 3-alkylthiophenes of formula 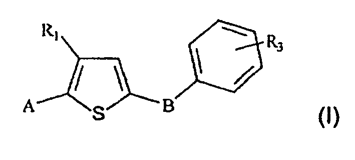 in which
A represents a CH₃, R₂CH₂, HOCH₂ or R₂CH(OH)- group,
which comprises:
(*a*) the reaction of a compound of formula 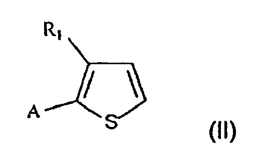 in which
A represents a CHO or R₂-CO- group,
with a compound of formula 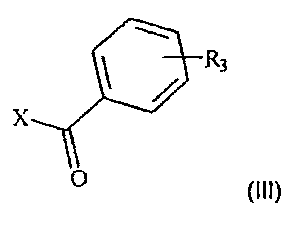 to give a compound of formula 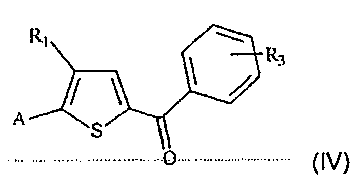 in which
A represents a CHO or R₂-CO group, and
(b) the reduction of the compound of formula IV thus obtained to give the compound of formula I.

4. Process according to Claim 1 in which the reaction mentioned in point (*a*) is performed with a compound of formula III, in which X represents halogen, in the presence of a Lewis acid and in a solvent chosen from chlorinated solvents and deactivated aromatic solvents, preferably with benzoyl chloride, in the presence of AlCl₃ and in methylene chloride, in which the molar ratio of compound III/Lewis acid/compound II is between 0.9-1.5/0.9-1.5/1 and preferably about 1/1/1.

5. Process according to Claim 1, in which the reduction mentioned in point (*b*) is performed by means of a single reductive treatment of the compound of formula 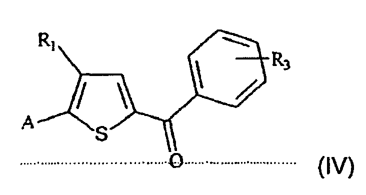 to give the compound of formula 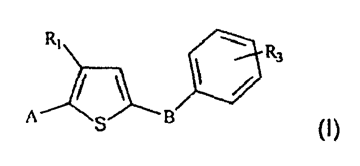 in which
A represents a CH₃ or R₂CH₂ group, and
B represents a CH₂ group.

6. Process according to Claim 5, in which the said reductive treatment is performed with sodium borohydride or sodium cyanoborohydride in the presence of trifluoroacetic acid.

7. Process according to Claim 1, in which the reduction mentioned in point (*b*) is performed by means of a first reduction reaction (*b*_{*1*}) of the compound of formula 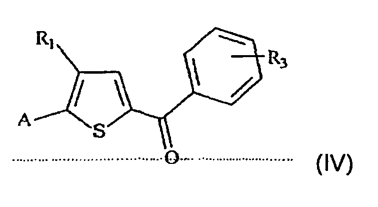 to give the hydroxylated intermediate compound of formula 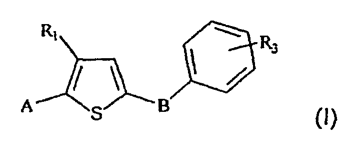 in which
A represents a CH₃, R₂CH₂, HOCH₂ or R₂CH(OH)- group, and
B represents a CHOH group;
optionally followed by a second reduction reaction (b₂) of the said hydroxylated intermediate of formula I to give a final compound of formula 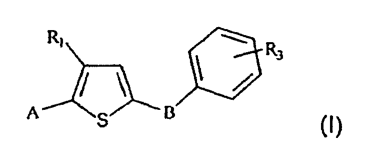 in which
A represents a CH₃ or R₂CH₂ group, and
B represents a CH₂ group.

8. Process according to Claim 7, in which the reduction mentioned in point (*b*) is performed by means of the said reduction reactions *(b*_{*1*}*)* and *(b*_{*2*}*)* successively.

9. Process according to Claim 7, in which the first of the said reduction reactions (*b*₁) is performed by treatment with metal hydrides, such as sodium borohydride, lithium aluminium hydride or boranes, or by treatment with aluminium isopropoxide, preferably by treatment with sodium borohydride.

10. Process according to Claim 7, in which the second of the said reduction reactions (*b*₂) is performed by treatment with a borohydride in the presence of a strong acid, such as trifluoroacetic acid, methanesulphonic acid or sulphuric acid, or with zinc iodide or by catalytic hydrogenation, preferably by treatment with sodium borohydride and trifluoroacetic acid.

11. Process according to Claim 7, in which the second of the said reduction reactions (*b*₂) is performed by catalytic hydrogenation of the hydroxylated intermediate of formula I (B=CHOH) dissolved in a suitable solvent, such as an alcohol, for instance methanol, ethanol or isopropanol, preferably methanol, or in a mixture of water and alcohols, at a hydrogen pressure of between 1 and 10 bar, at a temperature of between 15 and 60°C, in the presence of a hydrogenation catalyst chosen from palladium and platinum, preferably palladium supported on an inert support such as carbon, alumina, silica or zeolites, preferably on carbon, in a neutral or acidic medium.

12. Process according to Claim 7, in which the said hydroxylated intermediate compound of formula 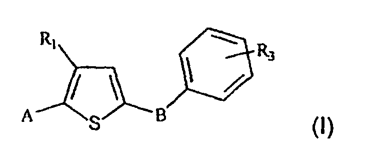 in which
A represents a CH₃, R₂CH₂, HOCH₂ or R₂CH(OH)- group, and
B represents a CHOH group
is purified by crystallization.

13. Process for preparing 2,3-dimethyl-5-benzylthiophene of formula 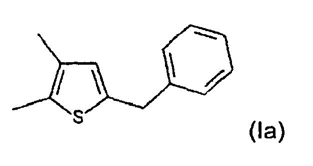 which comprises:
(*a*) the reaction of the compound of formula 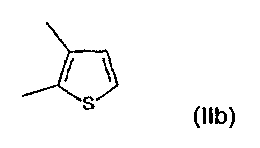 with a compound of formula 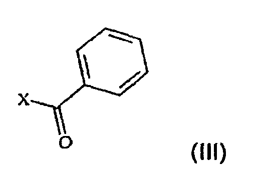 in which X represents halogen; to give the compound of formula 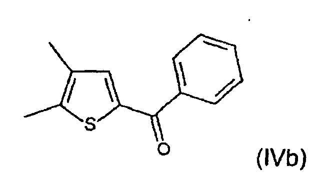
(*b*_{*1*}) the reduction of compound IVb to give the hydroxylated intermediate compound of formula 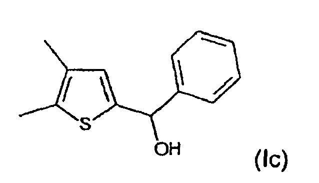 and
(*b*₂) the reduction of the hydroxylated intermediate compound Ic to give 2,3-dimethyl-5-benzylthiophene (Ia).

14. Process according to Claim 13, in which the reactions mentioned in points (*a*), (*b*₁) and (*b*₂) are performed under the experimental conditions of Claims 4, 9 and 10, respectively.

15. Process according to Claim 13, in which the hydroxylated intermediate compound of formula Ic is purified by crystallization, preferably from n-heptane.

## Patentansprüche

1. Verfahren zur Herstellung von 2,5-disubstituierten 3-Alkylthiophenen der Formel 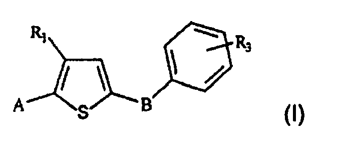 worin
A für eine CH₃-, R₂CH₂-, HOCH₂- oder R₂CH(OH)-Gruppe steht,
B für eine CHOH- oder CH₂-Gruppe steht,
R₁ für H oder für eine C₁-C₅-Alkylgruppe steht,
R₂ für eine C₁-C₅-Alkylgruppe steht,
R₃ für H oder eine C₁-C₅-Alkylgruppe oder eine C₁-C₅-Halogenalkylgruppe, oder ein unter Fluor, Chlor und Brom ausgewähltes Halogen steht,
bei dem man:
(a) eine Verbindung der Formel 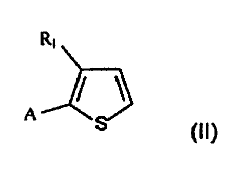 worin
A für eine CHO-, CH₃-, R₂CH₂- oder R₂-CO-Gruppe steht und
R₁ und R₂ die oben angegebenen Bedeutungen besitzen;
mit einer Verbindung der Formel 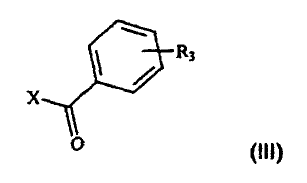 worin X für OH, Halogen oder eine Gruppe der Formel 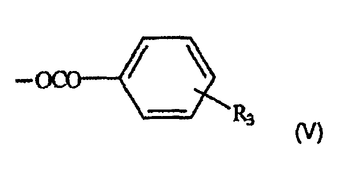 oder eine Gruppe der Formel
-OCOOR₄ (VI)
worin
R₄ für C₁-C₅-Alkyl, gegebenenfalls substituiertes Benzyl oder gegebenenfalls substituiertes Aryl steht und
R₃ die oben angegebenen Bedeutungen besitzt;
zu einer Verbindung der Formel 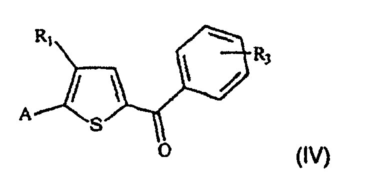 worin
A, R₁, R₂ und R₃ die oben angegebenen Bedeutungen besitzen, umsetzt und
(b) die so erhaltene Verbindung der Formel IV zu der Verbindung der Formel I reduziert.

2. Verfahren nach Anspruch 1 zur Herstellung von 2,5-disubstituierten 3-Alkylthiophenen der Formel 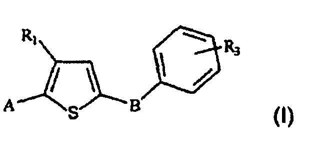 worin
A für eine CH₃- oder eine R₂CH₂-Gruppe steht,
bei dem man:
(a) eine Verbindung der Formel 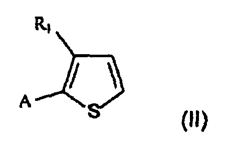 worin
A für eine CH₃- oder R₂CH₂-Gruppe steht,
mit einer Verbindung der Formel 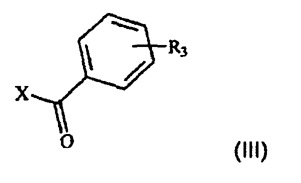 zu einer Verbindung der Formel 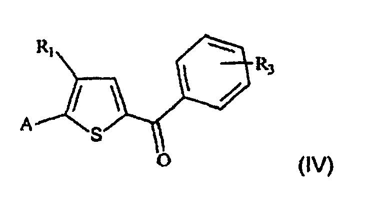 worin
A für eine CH₃- oder R₂CH₂-Gruppe steht, umsetzt und
(b) die so erhaltene Verbindung der Formel IV zu der Verbindung der Formel I reduziert.

3. Verfahren nach Anspruch 1 zur Herstellung von 2,5-disubstituierten 3-Alkylthiophenen der Formel 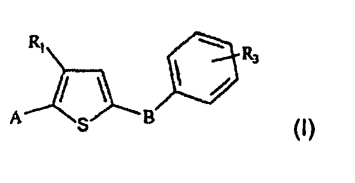 worin
A für eine CH₃-, R₂CH₂-, HOCH₂- oder R₂CH(OH)-Gruppe steht,
bei dem man:
(a) eine Verbindung der Formel 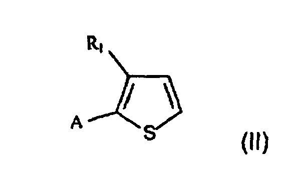 worin
A für eine CHO- oder R₂-CO-Gruppe steht,
mit einer Verbindung der Formel 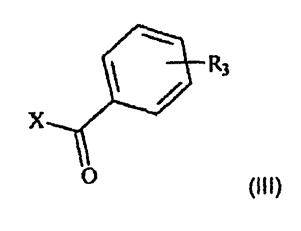 zu einer Verbindung der Formel 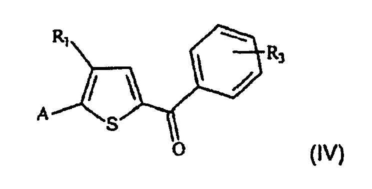 worin
A für eine CHO- oder R₂-CO-Gruppe steht, umsetzt und
(b) die so erhaltene Verbindung der Formel IV zu der Verbindung der Formel I reduziert.

4. Verfahren nach Anspruch 1, bei dem man die unter Punkt (a) erwähnte Umsetzung mit einer Verbindung der Formel III, worin X für Halogen steht, in Gegenwart einer Lewis-Säure und in einem unter chlorierten Lösungsmitteln und deaktivierten aromatischen Lösungsmitteln ausgewählten Lösungsmittel, vorzugsweise mit Benzoylchlorid, in Gegenwart von AlCl₃ und in Methylenchlorid, durchführt, wobei das Molverhältnis von Verbindung III/Lewis-Säure/Verbindung II zwischen 0,9-1,5/0,9-1,5/1 und vorzugsweise 1/1/1 liegt.

5. Verfahren nach Anspruch 1, bei dem man die unter Punkt (b) erwähnte Reduktion mittels einer einzigen reduktiven Behandlung der Verbindung der Formel 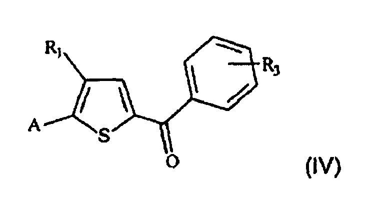 zu der Verbindung der Formel 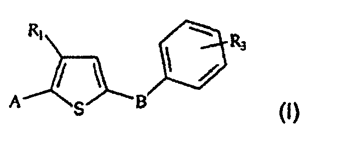 worin
A für eine CH₃- oder R₂CH₂-Gruppe steht und
B für eine CH₂-Gruppe steht,
durchführt.

6. Verfahren nach Anspruch 5, bei dem man die reduktive Behandlung mit Natriumborhydrid oder Natriumcyanoborhydrid in Gegenwart von Trifluoressigsäure durchführt.

7. Verfahren nach Anspruch 1, bei dem man die unter Punkt (b) erwähnte Reduktion mit Hilfe einer ersten Reduktionsreaktion (b₁) der Verbindung der Formel 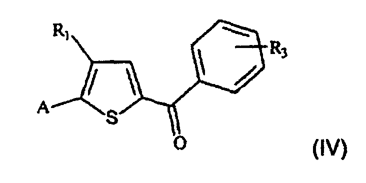 zu der hydroxylierten Zwischenverbindung der Formel 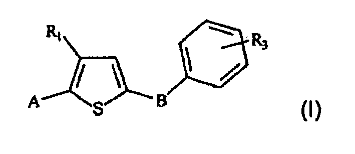 worin
A für eine CH₃-, R₂CH₂-, HOCH₂- oder R₂CH(OH)-Gruppe steht und
B für eine CHOH-Gruppe steht;
gegebenenfalls gefolgt von einer zweiten Reduktionsreaktion (b₂) des hydroxylierten Zwischenprodukts der Formel I zu einer Endverbindung der Formel 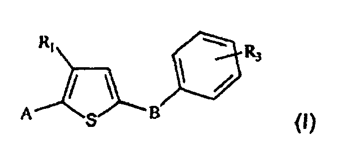 worin
A für eine CH₃- oder R₂CH₂-Gruppe steht und
B für eine CH₂-Gruppe steht,
durchführt.

8. Verfahren nach Anspruch 7, bei dem man die unter Punkt (b) erwähnte Reduktion mittels der Reduktionsreaktion (b₁) und (b₂) nacheinander durchfuhrt.

9. Verfahren nach Anspruch 7, bei dem man die erste der Reduktionsreaktionen (b₁) durch Behandlung mit Metallhydriden, wie Natriumborhydrid, Lithiumaluminiumhydrid oder Boranen, oder durch Behandlung mit Aluminiumisopropoxid, vorzugsweise durch Behandlung mit Natriumborhydrid, durchführt.

10. Verfahren nach Anspruch 7, bei dem man die zweite der Reduktionsreaktionen (b₂) durch Behandlung mit einem Borhydrid in Gegenwart einer starken Säure, wie Trifluoressigsäure, Methansulfonsäure oder Schwefelsäure, oder mit Zinkiodid oder durch katalytische Hydrierung, vorzugsweise durch Behandlung durch Natriumborhydrid und Trifluoressigsäure, durchführt.

11. Verfahren nach Anspruch 7, bei dem man die zweite der Reduktionsreaktionen (b₂) durch katalytische Hydrierung des in einem geeigneten Lösungsmittel, wie einem Alkohol, beispielsweise Methanol, Ethanol oder Isopropanol, vorzugsweise Methanol, oder in einem Gemisch aus Wasser und Alkoholen, gelösten hydroxylierten Zwischenprodukts der Formel I (B = CHOH) bei einem Wasserstoffdruck zwischen 1 und 10 bar, einer Temperatur zwischen 15 und 60°C in Gegenwart eines unter Palladium und Platin, vorzugsweise auf einem inerten Träger, wie Kohlenstoff, Aluminiumoxid, Siliziumdioxid oder Zeoliten, vorzugsweise auf Kohlenstoff, geträgertem Palladium, ausgewählten Hydrierkatalysators in neutralem oder sauren Medium durchführt.

12. Verfahren nach Anspruch 7, bei dem man die hydroxylierte Zwischenverbindung der Formel 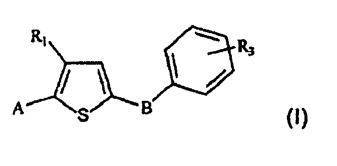 worin
A für eine CH₃-, R₂CH₂-, HOCH₂- oder R₂CH(OH)-Gruppe steht und
B für eine CHOH-Gruppe steht,
durch Kristallisation reinigt.

13. Verfahren zur Herstellung von 2,3-Dimethyl-5-benzylthiophen der Formel 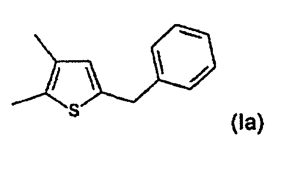 bei dem man:
(a) die Verbindung der Formel 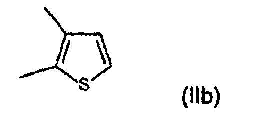 mit einer Verbindung der Formel 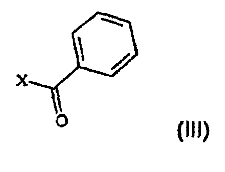 worin X für Halogen steht, zu der Verbindung der Formel 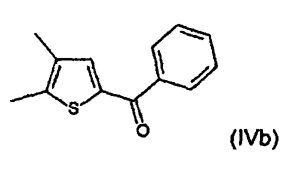 umsetzt;
(b₁) die Verbindung IVb zu der hydroxylierten Zwischenverbindung der Formel 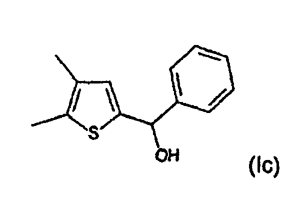 reduziert und
(b₂) die hydroxylierte Zwischenverbindung Ic zu 2,3-Dimethyl-5-benzylthiophen (Ia) reduziert.

14. Verfahren nach Anspruch 13, bei dem man die unter den Punkten (a), (b₁) und (b₂) erwähnten Umsetzungen unter den experimentellen Bedingungen der Ansprüche 4, 9 bzw. 10 durchführt.

15. Verfahren nach Anspruch 13, bei dem man die hydroxylierte Zwischenverbindung der Formel Ic durch Kristallisation, vorzugsweise aus n-Heptan, reinigt.

## Revendications

1. Procédé de préparation de 3-alkylthiophènes disubstitués en positions 2 et 5 de formule 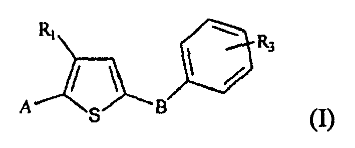 dans laquelle
A représente un groupe CH₃, R₂CH₂, HOCH₂ ou R₂CH(OH)-,
B représente un groupe CHOH ou CH₂,
R₁ représente H ou un groupe alkyle en C₁-C₅,
R₂ représente un groupe alkyle en C₁-C₅,
R₃ représente H ou un groupe alkyle en C₁-C₅ ou un groupe haloalkyle en C₁₋C₅, ou un halogène choisi parmi le fluor, le chlore et le brome,
qui comprend :
(a) la réaction d'un composé de formule 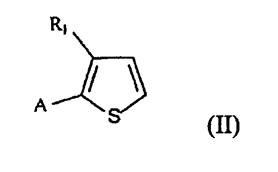 dans laquelle
A représente un groupe CHO, CH₃, R₂CH₂ ou R₂-CO-, et
R₁ et R₂ ont les significations données ci-dessus ;
avec un composé de formule 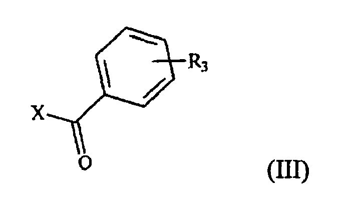 dans laquelle X représente OH, un halogène ou un groupe de formule 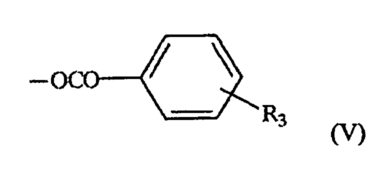 ou un groupe de formule
-OCOOR₄ (VI)
dans laquelle
R₄ représente un alkyle en C₁-C₅, un benzyle éventuellement substitué ou un aryle éventuellement substitué, et
R₃ a les significations ci-dessus ;
pour donner un composé de formule 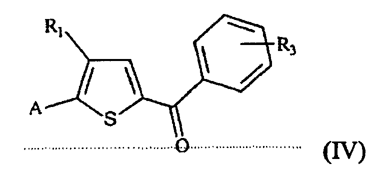 dans laquelle
A, R₁, R₂ et R₃ ont les significations données ci-dessus ; et
(b) la réduction du composé de formule IV ainsi obtenu pour donner le composé de formule I.

2. Procédé selon la revendication 1 de préparation de 3-alkylthiophènes disubstitués en positions 2 et 5 de formule 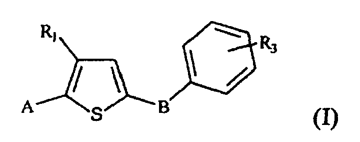 dans laquelle
A représente un groupe CH₃ ou R₂CH₂,
qui comprend :
(a) la réaction d'un composé de formule 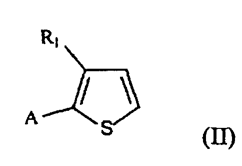 dans laquelle
A représente un groupe CH₃ ou R₂CH₂,
avec un composé de formule 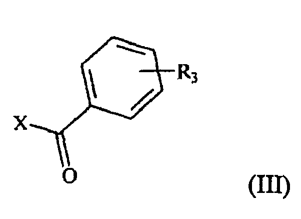 pour donner un composé de formule 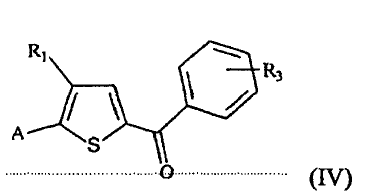 dans laquelle
A représente un groupe CH₃ ou R₂CH₂, et
(b) la réduction du composé de formule IV ainsi obtenu pour donner le composé de formule I.

3. Procédé selon la revendication 1 de préparation de 3-alkylthiophènes disubstitués en positions 2 et 5 de formule 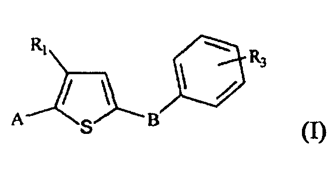 dans laquelle
A représente un groupe CH₃, R₂CH₂, HOCH₂ ou R₂CH(OH)-,
qui comprend :
(a) la réaction d'un composé de formule 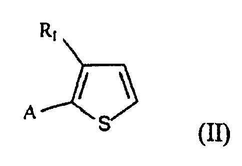 dans laquelle
A représente un groupe CHO ou R₂-CO-,
avec un composé de formule 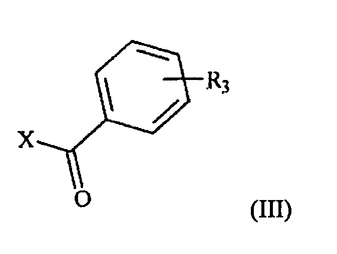 pour donner un composé de formule 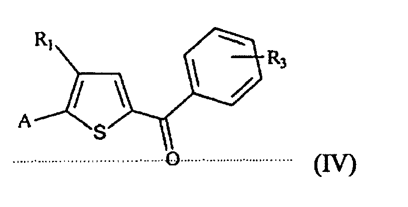 dans laquelle
A représente un groupe CHO ou R₂-CO-, et
(b) la réduction du composé de formule IV ainsi obtenu pour donner le composé de formule I.

4. Procédé selon la revendication 1 dans lequel la réaction mentionnée au point (a) est effectuée avec un composé de formule III, dans laquelle X représente un halogène, en présence d'un acide de Lewis et dans un solvant choisi parmi les solvants chlorés et les solvants aromatiques désactivés, de préférence avec du chlorure de benzoyle, en présence d'AlCl₃ et dans du chlorure de méthylène, où le rapport molaire du composé III/acide de Lewis/composé II est compris entre 0,9-1,5/0,9-1,5/1 et est de préférence d'environ 1/1/1.

5. Procédé selon la revendication 1, dans lequel la réduction mentionnée au point (b) est effectuée au moyen d'un seul traitement réducteur du composé de formule 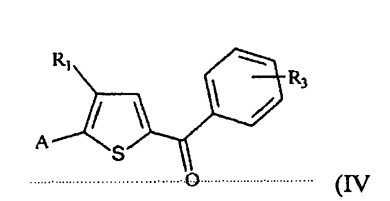 pour donner le composé de formule 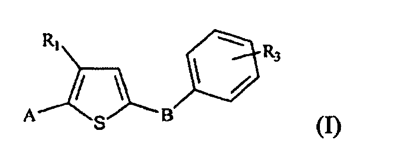 dans laquelle
A représente un groupe CH₃ ou R₂CH₂, et
B représente un groupe CH₂.

6. Procédé selon la revendication 5, dans lequel ledit traitement réducteur est effectué avec du borohydrure de sodium ou du cyanoborohydrure de sodium en présence d'acide trifluoroacétique.

7. Procédé selon la revendication 1, dans lequel la réduction mentionnée au point (b) est effectuée au moyen d'une première réaction de réduction (b₁) du composé de formule 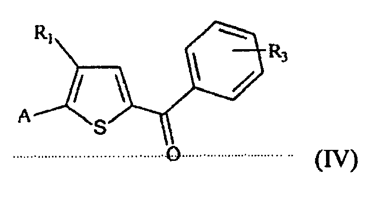 pour donner le composé intermédiaire hydroxylé de formule 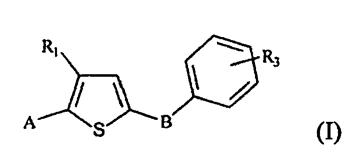 dans laquelle
A représente un groupe CH₃, R₂CH₂, HOCH₂ ou R₂CH(OH)-, et
B représente un groupe CHOH ;
éventuellement suivie d'une seconde réaction de réduction (b₂) dudit intermédiaire hydroxylé de formule I pour donner un composé final de formule 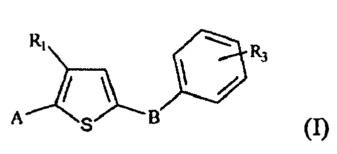 dans laquelle
A représente un groupe CH₃ ou R₂CH₂, et
B représente un groupe CH₂.

8. Procédé selon la revendication 7, dans lequel la réduction mentionnée au point (b) est effectuée au moyen desdites réactions de réduction (b₁) et (b₂) successivement.

9. Procédé selon la revendication 7, dans lequel la première desdites réactions de réduction (b₁) est effectuée par traitement avec des hydrures métalliques, tels que le borohydrure de sodium, l'hydrure de lithium-aluminium ou des boranes, ou par traitement avec de l'isopropoxyde d'aluminium, de préférence par traitement avec du borohydrure de sodium.

10. Procédé selon la revendication 7, dans lequel la seconde desdites réactions de réduction (b₂) est effectuée par traitement avec un borohydrure en présence d'un acide fort, tel que l'acide trifluoroacétique, l'acide méthanesulphonique ou l'acide sulfurique, ou avec de l'iodure de zinc ou par hydrogénation catalytique, de préférence par traitement avec du borohydrure de sodium et de l'acide trifluoroacétique.

11. Procédé selon la revendication 7, dans lequel la seconde desdites réactions de réduction (b₂) est effectuée par hydrogénation catalytique de l'intermédiaire hydroxylé de formule I (B = CHOH) dissous dans un solvant approprié, tel qu'un alcool, par exemple le méthanol, l'éthanol ou l'isopropanol, de préférence le méthanol, ou dans un mélange d'eau et d'alcools, avec une pression d'hydrogène comprise entre 1 et 10 bar, à une température comprise entre 15 et 60°C, en présence d'un catalyseur d'hydrogénation choisi parmi le palladium et le platine, de préférence du palladium supporté sur un support inerte tel que du carbone, de l'alumine, de la silice ou des zéolites, de préférence sur du carbone, dans un milieu neutre ou acide.

12. Procédé selon la revendication 7, dans lequel ledit composé intermédiaire hydroxylé de formule 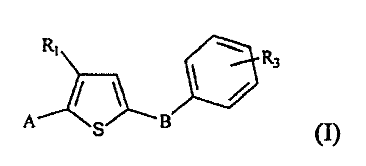 dans laquelle
A représente un groupe CH₃, R₂CH₂, HOCH₂ ou R₂CH(OH)-, et
B représente un groupe CHOH
est purifié par cristallisation.

13. Procédé de préparation du 2,3-diméthyl-5-benzylthiophène de formule 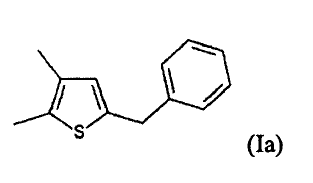 qui comprend :
(a) la réaction du composé de formule 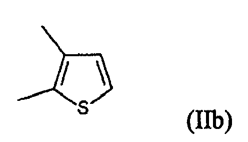 avec un composé de formule 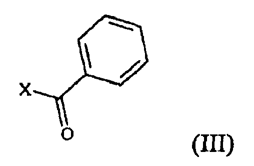 dans laquelle
X représente un halogène ;
pour donner le composé de formule 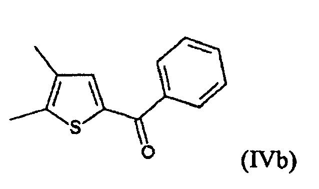
(b₁) la réduction du composé IVb pour donner le composé intermédiaire hydroxylé de formule 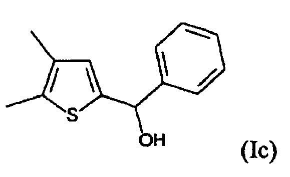 et
(b₂) la réduction du composé intermédiaire hydroxylé Ic pour donner le 2,3-diméthyl-5-benzylthiophène (Ia).

14. Procédé selon la revendication 13, dans lequel les réactions mentionnées aux points (a), (b₁) et (b₂) sont effectuées dans les conditions expérimentales des revendications 4, 9 et 10, respectivement.

15. Procédé selon la revendication 13, dans lequel le composé intermédiaire hydroxylé de formule Ic est purifié par cristallisation, de préférence dans le n-heptane.
